# EUROPÄISCHE PATENTSCHRIFT

(11) **EP 0 965 337 B1**
(45) Veröffentlichungstag und Bekanntmachung des Hinweises auf die Patenterteilung: **28.07.2004**
(21) Anmeldenummer: 99114947.7
(22) Anmeldetag: 08.05.1995
(51) Int. Cl.: A61K 9/20

(54) **Verfahren zur Herstellung von Vorrichtungen zur kontrollierten Freisetzung von Wirkstoffen**
Method to produce devices for controlled drug release
Procéde de préparation de dispositifs pour la libération contrôlée de medicaments

(30) Priorität: 13.05.1994 DE 4416926
(43) Veröffentlichungstag der Anmeldung: 22.12.1999
(62) Teilanmeldung aus: 95920805.9
(73) Patentinhaber: LTS LOHMANN Therapie-Systeme GmbH, 56567 Neuwied (DE)
(72) Erfinder: Cremer, Karsten, 53119 Bonn (DE)
(74) Vertreter: Flaccus, Rolf-Dieter, Dr.

(56) Entgegenhaltungen:
- WO-A-94/07470
- DATABASE WPI Section Ch, Week 9715 Derwent Publications Ltd., London, GB; Class B07,Page 33, AN 97-154886 XP002055483 & CN 1 091 003 A (YANG, J.), 24. August 1994 (1994-08-24)

## Beschreibung

Die Erfindung bezieht sich auf ein Verfahren zur Herstellung von Vorrichtungen zur kontrollierten Freisetzung von Wirkstoffen, bestehend aus einer wirkstoffhaltigen Matrix und einer die Matrix mindestens teilweise bedeckenden erodierbaren Feststoffmasse, wobei die Dicke der erodierbaren Masse über deren Erstreckung durch Dickegradienten bestimmt ist.

Vorrichtungen dieser Art sind in der Patentanmeldung DE-A-4341442 beschrieben. Sie dienen dazu, Wirkstoffe in flüssigen Medien, wie z. B. Körperflüssigkeiten kontrolliert, d. h. mit vorgegebenem Geschwindigkeitsprofil freizusetzen. Ein wichtiges Einsatzgebiet dieser Vorrichtungen ist die perorale Verabreichung von Arzneistoffen. Hier ist häufig das Ziel, Wirkstoffe mit kurzer biologischer Halbwertszeit so zu verabreichen, daß bei geringer Einnahmefrequenz gleichmäßige Plasmakonzentrationen aufrechterhalten werden

Das Geschwindigkeitsprofil der Wirkstofffreisetzung wird bei diesen Vorrichtungen insbesondere von den Dickegradienten der erodierbaren Feststoffmasse mitbestimmt, welche die wirkstoffhaltige Matrix zunächst zumindest teilweise bedecken. Im Laufe der Freisetzung kommt es zu einer voranschreitenden Erosion dieser Feststoffmasse, wodurch sich in Abhängigkeit von ihren Dickegradienten die Kontaktfläche zwischen der wirkstoffhaltigen Matrix und dem flüssigen Medium, in welchem sich die Vorrichtung befindet, vergrößert. Mittels dieses Effektes läßt sich bei entsprechender Wahl der Dickegradienten beispielsweise eine relativ konstante Freisetzungsgeschwindigkeit aufrechterhalten, wie es bei Retardarzneimitteln häufig erwünscht ist.

Aufgrund des neuartigen Konstruktionsprinzips finden sich im Stand der Technik bisher kaum Angaben zur Herstellungsmöglichkeiten für Vorrichtungen dieser Art. Lediglich in der WO 94/07470 wird die Erzeugung von erodierbaren Überzügen unterschiedlicher Dicke durch herkömmliche Beschichtungstechniken in der Rolltrommel oder durch Sprüh- oder Wirbelschichttechniken erwähnt. Diese Techniken sind jedoch nicht allgemein anwendbar und zur einfachen Erzeugung einer wirkstoffhaltigen Matrix mit einer erodierbaren Feststoffmasse mit Dickegradienten nicht optimal. Der Erfindung liegt daher die Aufgabe zugrunde, eine verbesserte Verfahrensweise zur Herstellung der oben genannten Vorrichtungen vorzusehen.

Diese Aufgabe wird erfindungsgemäß dadurch gelöst, daß die wirkstoffhaltige Matrix und die erodierbare Masse in einem kontinuierlichen Prozeß als bahn-, band- oder strangförmiges Material hergestellt wird und daraus durch Schneiden oder Stanzen Vorrichtungen zur kontrollierten Freisetzung von Wirkstoffen gewonnen werden. Erfindungsgemäß werden also die wirkstoffhaltige Matrix und die erodierbare Masse in einem Prozeßschritt geformt und zusammengebracht. Dabei gibt es mehrere verfahrenstechnische Prozesse, die zu dieser Lösung beitragen können.

Das erfindungsgemäße Verfahren ist insbesondere zweckmäßig, wenn der Wirkstoff oder andere notwendige Komponenten der Vorrichtung nicht tablettierbar sind, wie es bei flüssigen Substanzen oder bei Festsubstanzen mit niedrigem Schmelzpunkt der Fall ist, oder wenn aus einem anderen Grund auf die Tablettierung verzichtet werden soll, da die Formung der wirkstoffhaltigen Matrix und/oder der erodierbaren Feststoffmasse nun durch einen alternativen Prozeß erfolgen kann. Bei Verwendung von thermoplastischen Ausgangsmaterialien bieten sich solche Prozesse an, welche die Thermoplastizität ausnutzen und die Materialien durch Schmelzen, Gießen, Injizieren oder Extrudieren formen.

Erfindungsgemäß wird man dabei häufig bevorzugen, die wirkstoffhaltige Matrix zunächst in Form eines bahn-, band- oder strangformigen Körpers bereitzustellen. In dieses Material lassen sich - gegebenenfalls im noch warmen Zustand - durch Stempeln oder Walzen Vertiefungen bzw. Dickegradienten einbringen, die im anschließenden Zusammenbringen mit der erodierbaren Feststoffmasse die Dickegradienten der letzteren im wesentlichen mitbestimmen. Vorrichtungen der o. g. Art können anschließend durch Schneiden oder Stanzen ausgeeinzelt werden.

Ein ähnliches Vorgehen ist erfindungsgemäß jedoch auch dann möglich, wenn die Ausgangsmaterialien keine ausgeprägten thermoplastischen Eigenschaften besitzen. In diesem Fall kann die Formgebung auch unter Zuhilfenahme einer flüssigen Phase, also durch Verarbeiten einer Lösung, eines Gels, einer Suspension oder Paste erfolgen. Mit Lösungen und Suspensionen lassen sich beispielsweise Trägerfolien beschichten und durch anschließende Trocknung bahnförmige Körper erzeugen. Diese sind analog zu den durch thermoplastische Formgebung erzeugten Körpern weiterzuverarbeiten. Ebenfalls möglich ist das Anteigen von pulverförmigen Materialien bis zur pastösen Konsistenz, aus der die wirkstoffhaltige Matrix durch Extrusion gewonnen und wie beschrieben weiterverarbeitet wird.

Ein erfindungsgemäßes Verfahren zur Herstellung von Vorrichtungen der o. g. Art kann auch die Kombination von Prozeßschritten verschiedener Art beinhalten. So kann es beispielsweise vorteilhaft sein, zunächst die wirkstoffhaltige Matrix durch einen oder mehrere hintereinandergeschaltete Beschichtungsprozesse herzustellen, in diese dann durch Stempel Vertiefungen zu prägen und anschließend in die Vertiefungen die erodierbare Masse in geschmolzenem Zustand zu injizieren.

Es kann sich als notwendig erweisen, vor dem Zusammenbringen der wirkstoffhaltigen Matrix mit der erodierbaren Masse einen oder mehrere adhäsionsfördernde Zusätze auf die Kontaktfläche zwischen den einzelnen Komponenten der Vorrichtung aufzutragen. Die Auftragung der klebenden Hilfsstoffe geschieht vorzugsweise dadurch, daß eine Lösung dieser Stoffe vorliegt, die in einem Beschichtungs-, Sprüh- oder Tauchverfahren appliziert wird.

Alternativ zu den oben beschriebenen Ausgestaltungen des Verfahrens kann auch eine simultane Formung der wirkstoffhaltigen Matrix und der erodierbaren Feststoffmasse, und ihr Zusammenbringen, in einem Prozeßschritt erfolgen. Diese Art der Herstellung bietet sich insbesondere dann an, wenn sich die wirkstoffhaltige Matrix und die erodierbare Feststoffmasse in annähernd gleichen Prozessen und bei annähernd gleichen Prozeßbedingungen fertigen lassen. Lassen sich beispielsweise alle Komponenten der herzustellenden Vorrichtung durch eine thermoplastische Formgebung bei gleicher oder ähnlicher Temperatur fertigen, so ist die Koextrusion der Komponenten ein bevorzugtes Verfahren.

Unabhängig von der Ausgestaltung des Herstellungsverfahrens gelten alle beschriebenen Optionen auch für den Fall, daß Vorrichtungen zur kontrollierbaren Freisetzung von Wirkstoffen mit mehr als einer wirkstoffhaltigen Matrix und einer die Matrices zumindest teilweise bedeckenden, erodierbaren Feststoffmasse hergestellt werden sollen.

## Patentansprüche

1. Verfahren zur Herstellung von Vorrichtungen zur kontrollierten Freisetzung von Wirkstoffen, bestehend aus einer wirkstoffhaltigen Matrix und einer die Matrix mindestens teilweise bedeckenden erodierbaren Feststoffmasse, wobei die Dicke der erodierbaren Masse über deren Erstreckung durch Dickegradienten bestimmt ist, **dadurch gekennzeichnet, daß** die wirkstoffhaltige Matrix und die erodierbare Masse in einem kontinuierlichen Prozeß als bahn-, band- oder strangförmiges Material hergestellt und daraus durch Schneiden oder Stanzen Vorrichtungen zur kontrollierten Freisetzung von Wirkstoffen gewonnen werden.

2. Verfahren nach Anspruch 1, **dadurch gekennzeichnet, daß** die wirkstoffhaltige Matrix zusätzlich durch Stempeln oder Walzen eingebrachte Vertiefungen besitzt.

3. Verfahren nach Anspruch 1, **dadurch gekennzeichnet, daß** die wirkstoffhaltige Matrix und/oder die erodierbare Masse aus thermoplastischem Material in einem Schmelz-, Gieß-, Injektions- oder Extrusionsverfahren geformt wird.

4. Verfahren nach Anspruch 3, **dadurch gekennzeichnet, daß** die wirkstoffhaltige Matrix und die erodierbare Masse koextrudiert werden.

5. Verfahren nach Anspruch 1, **dadurch gekennzeichnet, daß** die wirkstoffhaltige Matrix und/oder die erodierbare Masse unter Zuhilfenahme einer flüssigen Phase aus einer Lösung, Suspension, Paste oder Gel geformt und fallweise die flüssige Phase verdampft wird.

6. Verfahren nach einem oder mehreren der Ansprüche 1 bis 5, **dadurch gekennzeichnet, daß** die Vorrichtung zur kontrollierten Freisetzung von Wirkstoffen mit mehr als einer wirkstoffhaltigen Matrix hergestellt wird.

7. Verfahren nach einem oder mehreren der Ansprüche 1 bis 6, **dadurch gekennzeichnet, daß** vor dem Zusammenbringen von wirkstoffhaltiger Matrix und erodierbarer Masse ein adhäsionsfördernder Hilfsstoff auf die Kontaktfläche zwischen den beiden Komponenten aufgebracht wird

## Claims

1. Process for the production of devices for the controlled release of active substances, made up of an active substance-containing matrix and an erodible mass of solids at least partially covering said matrix, the thickness of said erodible mass across its extension being determined by thickness gradients, **characterized in that** the active substance-containing matrix and the erodible mass are produced in a continuous process as web-, tape- or strand-shaped material, and that from this material are obtained devices for the controlled release of active substances by cutting or punching

2. Process according to claim 1, **characterized in that** the active substance-containing matrix in addition has depressions which have been introduced by stamping or rolling.

3. Process according to claim 1, **characterized in that** the active substance-containing matrix and/or the erodible mass is formed from thermoplastic material in a melting, casting, injection or extrusion process.

4. Process according to claim 3, **characterized in that** the active substance-containing matrix and the erodible mass are coextruded.

5. Process according to claim 1, **characterized in that** the active substance-containing matrix and/or the erodible mass is formed from a solution, suspension, paste or gel with the aid of a liquid phase, and that, according to circumstances, said liquid phase is optionally evaporated.

6. Process according to one or more of claims 1 to 5, **characterized in that** the said device is produced for the controlled release of active substances with more than one active substance-containing matrix.

7. Process according to one or more of claims 1 to 6, **characterized in that** before the active substance-containing matrix is combined with the erodible mass, an adhesion-promoting auxiliary substance is applied to the contact surface between the two components.

## Revendications

1. Procédé de fabrication de dispositifs de libération contrôlée de produits actifs, constitués d'une matrice qui contient des produits actifs et d'une masse solide érodable qui recouvre la matrice au moins en partie, l'épaisseur de la masse érodable et son extension étant déterminées par des gradients d'épaisseur, **caractérisé en ce que** la matrice qui contient le produit actif et la masse érodable sont fabriquées en une opération continue sous la forme d'un matériau en forme de nappe, de bandes ou de barreaux et les dispositifs de libération contrôlée de produits actifs étant obtenus par découpe ou estampage de ce matériau.

2. Procédé selon la revendication 1, **caractérisé en ce que** la matrice qui contient le produit actif possède en outre des creux qui sont ménagés par poinçonnage ou laminage.

3. Procédé selon la revendication 1, **caractérisé en ce que** la matrice qui contient le ou les produits actifs et/ou la masse érodable sont façonnées à partir d'un matériau thermoplastique dans un procédé de fusion, de coulée, d'injection ou d'extrusion.

4. Procédé selon la revendication 3, **caractérisé en ce que** la matrice qui contient le ou les produits actifs et la masse érodable sont coextrudées.

5. Procédé selon la revendication 1, **caractérisé en ce que** la matrice qui contient le ou les produits actifs et/ou la masse érodable sont façonnées à partir d'une phase liquide constituée d'une solution, d'une suspension, d'une pâte ou d'un gel et la phase liquide étant éventuellement évaporée.

6. Procédé selon l'une ou plusieurs des revendications 1 à 5, **caractérisé en ce que** le dispositif de libération contrôlée de produits actifs est préparé avec plus d'une matrice qui contiennent le ou les produits actifs.

7. Procédé selon l'une ou plusieurs des revendications 1 à 6, **caractérisé en ce qu'**avant l'assemblage de la matrice qui contient le ou les produits actifs et de la masse érodable, une substance auxiliaire qui favorise l'adhérence des deux composants est appliquée sur la surface de contact entre les deux composants.
